(19)

Europäisches
Patentamt
European
Patent Office
Office européen
des brevets

(11) **EP 4 070 806 A1**

(12) **EUROPEAN PATENT APPLICATION**
published in accordance with Art. 153(4) EPC

(43) Date of publication:
**12.10.2022 Bulletin 2022/41**

(21) Application number: **20896562.4**

(22) Date of filing: **26.11.2020**

(51) International Patent Classification (IPC):
$A61K\ 35/747^{(2015.01)}$  $A23L\ 33/135^{(2016.01)}$
$A61K\ 31/702^{(2006.01)}$  $A61K\ 31/716^{(2006.01)}$
$A61K\ 31/721^{(2006.01)}$  $A61K\ 31/729^{(2006.01)}$
$A61K\ 31/731^{(2006.01)}$  $A61K\ 31/732^{(2006.01)}$
$A61K\ 31/733^{(2006.01)}$  $A61K\ 31/734^{(2006.01)}$
$A61K\ 31/736^{(2006.01)}$  $A61K\ 36/02^{(2006.01)}$
$A61P\ 9/02^{(2006.01)}$  $A61P\ 43/00^{(2006.01)}$

(52) Cooperative Patent Classification (CPC):
A23L 33/135; A61K 31/702; A61K 31/716;
A61K 31/721; A61K 31/729; A61K 31/731;
A61K 31/732; A61K 31/733; A61K 31/734;
A61K 31/736; A61K 35/747; A61K 36/02;
A61P 9/02; A61P 43/00

(86) International application number:
**PCT/JP2020/044053**

(87) International publication number:
**WO 2021/111980 (10.06.2021 Gazette 2021/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.12.2019 JP 2019218216**

(71) Applicant: **Life Quality Institute, Inc.
Tokyo, 101-0053 (JP)**

(72) Inventor: **NAGATA, Katsutaro
Tokyo 101-0053 (JP)**

(74) Representative: **Osha Liang
2, rue de la Paix
75002 Paris (FR)**

(54) **PROPHYLACTIC OR AMELIORATING AGENT FOR ORTHOSTATIC HYPOTENSION, AND COMPOSITION CONTAINING SAME**

(57)     The present invention aims to provide an agent for preventing or ameliorating orthostatic hypotension, which agent is highly safe and effective, causes less side effects, and can be continuously used for a long time.

One embodiment of the present invention is an agent for preventing or ameliorating orthostatic hypotension comprising, as an active ingredient, a lactic acid bacterium, Lactobacillus delbrueckii subspecies lactis or a lactic acid bacterium-derived ingredient therefrom.

Figure 1: Changes in ΔSBP for each group before and after administration

EP 4 070 806 A1

**Description**

TECHNICAL FIELD

The present invention relates to an agent and a composition for preventing, treating or ameliorating orthostatic hypotension, and more specifically relates to an agent for preventing, treating or ameliorating orthostatic hypotension comprising a lactic acid bacterium and/or a lactic acid bacterium-derived ingredient as an active ingredient, and to a composition comprising the same.

BACKGROUND ART

Orthostatic hypotension is an excessive drop in blood pressure that occurs during a change of body position from lying to standing. Orthostatic hypotension is defined as a drop in systolic blood pressure (SBP) greater than 20mmHg after standing up. The main symptoms are: 1. orthostatic dizziness and vertigo, 2. discomfort after standing, 3. discomfort after bathing, 4. palpitations and shortness of breath, and 5. difficulty getting up in the morning. These are considered as the five major symptoms. In addition, some patients may experience falls, fainting, and generalized convulsions.

The etiology of orthostatic hypotension may be neurological, cardiovascular, or drugs, and is often constitutional or hereditary. More specifically, the afferent, central, or efferent portion of the autonomic reflex arch may be impaired by a disease or a drug, myocardial contractility or vascular reactivity may be reduced, circulating blood volume may be decreased, or hormonal responses may be defective. In such cases, homeostatic mechanisms may be insufficient and unable to restore the dropped blood pressure. Diseases that cause orthostatic hypotension include idiopathic orthostatic hypotension (IOH), postprandial hypotension, postural tachycardia syndrome (POT), multiple system atrophy (Shy-Drager syndrome), Parkinson's disease, stroke, amyloidosis, Guillain-Barre syndrome, diabetic neuropathy, alcoholic neuropathy, nutritional neuropathy, familial autonomic imbalance (Riley-Day syndrome), adrenal insufficiency, heart failure, myocardial infarction, and peripheral venous insufficiency. Further, drugs that cause orthostatic hypotension include antihypertensive drugs, psychotropic drugs, antipsychotics, alcohol, and diuretics.

The diagnosis of orthostatic hypotension is based on subjective symptoms and when three or more out of the five major symptoms of orthostatic hypotension (1. orthostatic dizziness and vertigo, 2. discomfort after standing, 3. discomfort after bathing 4. palpitations and shortness of breath, and 5. difficulty getting up in the morning) are experienced, orthostatic hypotension is suspected. More detailed examination can be done by means of the Head-Up Tilt test. This test is used to monitor blood pressure changes associated with changing body position from lying to standing, and the blood pressure can be observed to see if a drop in systolic blood pressure of 20mmHg or more occurs by the position change from a supine position to an upright position. Diastolic blood pressure changes may increase in healthy subjects after standing, but may increase or decrease in patients.

Currently, drugs are primarily used to treat orthostatic hypotension. Midodrine, for example, is a peripheral alpha agonist that constricts both arteries and veins and is often effective in orthostatic hypotension. However, this drug has adverse effects, such as paresthesia and pruritus. This drug is also not recommended for patients with coronary or peripheral arterial disease. Amezinium metilsulfate may be effective in orthostatic hypotension, because it increases sympathetic function and improves circulation dynamics by inhibiting noradrenaline inactivation. However, because this drug stimulates the sympathetic nervous system, it may cause hyperthyroidism, increased intraocular pressure, and dysuria. Although these drugs are commonly used, they are all hypertensive agents, are also drugs with many side effects, and are ineffective in many cases. Furthermore, these drugs cannot be used continuously for prolonged periods of time because of the tachycardia that occurs with prolonged continuous use.

Fludrocortisone, a mineral corticoid, often alleviates symptoms by causing sodium retention and thereby increasing plasma volume. However, fludrocortisone is effective only when sodium intake is sufficient and can only be used for special pathologies, such as multiple system atrophy (Shy-Drager syndrome). The drug may also improve the responsiveness of peripheral vasoconstriction to sympathetic stimulation, but supine hypertension, cardiac failure, and hypokalemia may occur.

Treatments other than drugs include adequate fluid and salt intake, limitation of carbohydrates and alcohol consumption, moderate exercise, devising recumbency positions, and wearing an abdominal bandage or elastic stockings, and these are considered to be effective. However, the effectiveness of these methods also varies greatly from person to person and is not reliable.

No new drugs have been available for the treatment of orthostatic hypotension for the past 30 years. In addition, as mentioned above, there are drawbacks to all conventional treatment methods. Therefore, it is hoped that new drugs that are safe and effective will emerge.

In recent years, functional lactic acid bacteria have attracted attention in the maintenance and improvement of health, various strains have been isolated and the utility of these strains are being investigated. Lactobacillus delbrueckii subspecies lactis KLAB-4 strain (Lactobacillus delbrueckii subsp. lactis KLAB-4) is a strain isolated from fermented milk, and has been deposited to National Institute of Technology and Evolution (2-5-8, Kazusakamatari, Kisarazu-shi, Chiba, Japan) as a novel lactic acid bacterium belonging to genus Lactobacillus, species delbrueckii, subspecies lactis (Accession No. NITE BP-394; the national deposition strain of August 9, 2007 of the original deposition date was transferred to the International Deposition under the Budapest Treaty (September 22, 2008)). This lactic acid bacterium has been known to have an excellent anti-allergic function, and further an anti-autoimmune disease function, diabetes-improving function, and hypoglycemia-improving function (Patent Documents 1 and 2, Non-Patent Document 1).

PRIOR ART DOCUMENTS

PATENT DOCUMENTS

Patent Document 1: Japanese Patent Gazette No. 5554994
Patent Document 2: Japanese Patent Gazette No. 6747724

NON-PATENT DOCUMENT

Non-Patent Document 1: "Health functions of lactic acid bacteria (Lactic Acid Bacteria and Human Health)," Airo Tategaki, Comprehensive Medicine, 17(1), 8-19 (2018)

SUMMARY OF THE INVENTION

PROBLEMS TO BE SOLVED BY THE INVENTION

An object of the present invention is to provide an agent for preventing or ameliorating orthostatic hypotension, which agent is highly safe and effective, causes less side effects, and can be continuously used for a long time.

MEANS FOR SOLVING THE PROBLEM

The inventor of the present invention diligently studied to solve the above problem, as a result, found that a specific lactic acid bacterium shows an excellent preventing or ameliorating effect on orthostatic hypotension, and completed the present invention.
According to the present invention, the followings are provided:

[1] An agent for preventing or ameliorating orthostatic hypotension comprising, as an active ingredient, a lactic acid bacterium Lactobacillus delbrueckii subspecies lactis or a lactic acid bacterium-derived ingredient therefrom;
[2] The agent for preventing or ameliorating orthostatic hypotension according to the above described [1], wherein said lactic acid bacterium comprises a lactic acid bacterium selected from the group consisting of Lactobacillus delbrueckii subspecies lactis KLAB-4 strain (NITE BP-394) and variants thereof;
[3] The agent for preventing or ameliorating orthostatic hypotension according to the above described [1] or [2], which further comprises a water-soluble dietary fiber ingredient, or which is used in combination with a water-soluble dietary fiber ingredient;
[4] The agent for preventing or ameliorating orthostatic hypotension according to the above described [3], wherein said water-soluble dietary fiber ingredient is selected from the group consisting of glucomannan, pectin, guar gum, alginic acid, polydextrose, β-glucan, fructan, inulin, levan, graminan, gum arabic, maltitol, psyllium, indigestible oligosaccharide, indigestible dextrin, agarose, sodium alginate, carrageenan, fucoidan, porphyran, laminaran, seaweed, and agar;
[5] The agent for preventing or ameliorating orthostatic hypotension according to the above described [4], wherein said water-soluble dietary fiber ingredient is glucomannan;
[6] A composition comprising the agent for preventing or ameliorating orthostatic hypotension according to any one of the above described [1] to [5];
[7] A pharmaceutical composition comprising the agent for preventing or ameliorating orthostatic hypotension according to any one of the above described [1] to [5];
[8] A food or drink composition comprising the agent for preventing or ameliorating orthostatic hypotension according to any one of the above described [1] to [5];
[9] A feed composition for animal or a pharmaceutical composition for animal comprising the agent for preventing or ameliorating orthostatic hypotension according to any one of the above described [1] to [5];

[10] A method for preventing or ameliorating orthostatic hypotension, comprising administering the agent for preventing or ameliorating orthostatic hypotension according to any one of the above described [1] to [5], or the composition according to any one of the above described [6] to [9], to a subject.

EFFECT OF THE INVENTION

The agent for preventing or ameliorating orthostatic hypotension of the present invention, and the composition comprising the same can effectively ameliorate orthostatic hypotension. Since the agent for preventing or ameliorating orthostatic hypotension and the composition comprising the same of the present invention comprise a lactic acid bacterium having high safety or a lactic acid bacterium-derived ingredient therefrom as an active ingredient, they are free from worry about side effects and highly safe even after administration or intake for a long term. Further, a water-soluble dietary fiber ingredient, for example, glucomannan is also a beneficial ingredient which human beings have eaten for a long time, and is highly safe. Therefore, the agent for preventing or ameliorating orthostatic hypotension of the present invention and the composition comprising the same can be used for prevention and can also be used for treatment.

BRIEF DESCRIPTION OF THE DRAWINGS

[Figure 1]

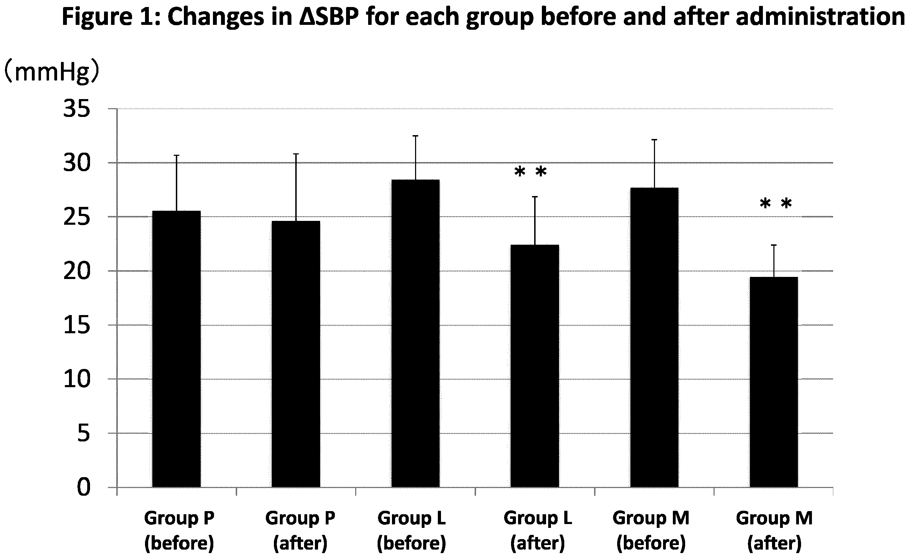

Figure 1 is a figure which shows the changes in $\Delta$SBP for each group before and after administration. The "**" represents a $p < 0.01$ as compared with the value before the administration.
[Figure 2]

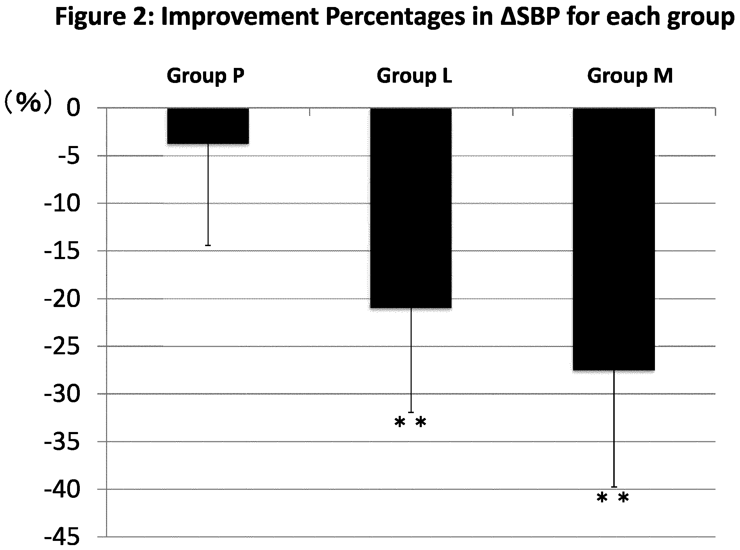

Figure 2 is a figure which shows the percentage improvement (%) in $\Delta$SBP for each group. The "**" represents a $p < 0.01$ as compared with Group P.
[Figure 3]

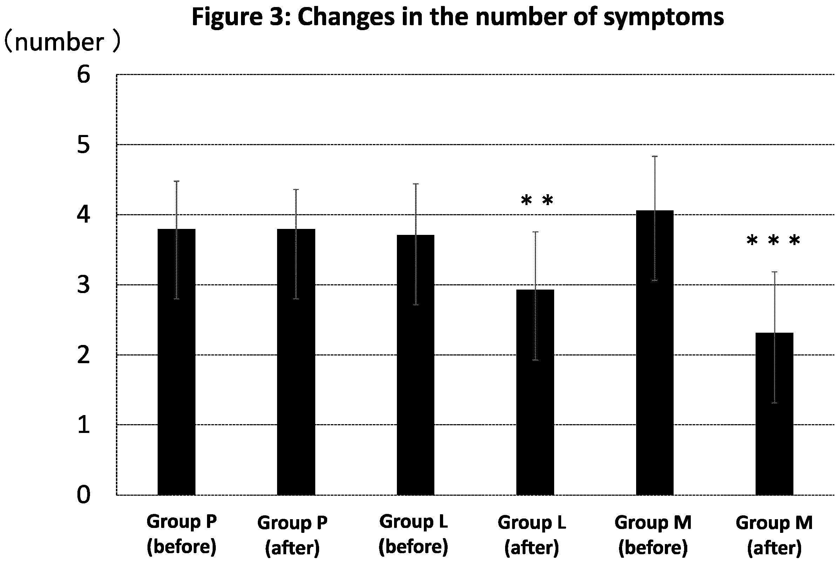

Figure 3 is a figure which shows the changes in the number of symptoms in each group compared before and after administration. The "**" represents a $p < 0.01$ as compared with the previous value, and the "***" represents a $p < 0.01$ as compared with the previous value.
[Figure 4]

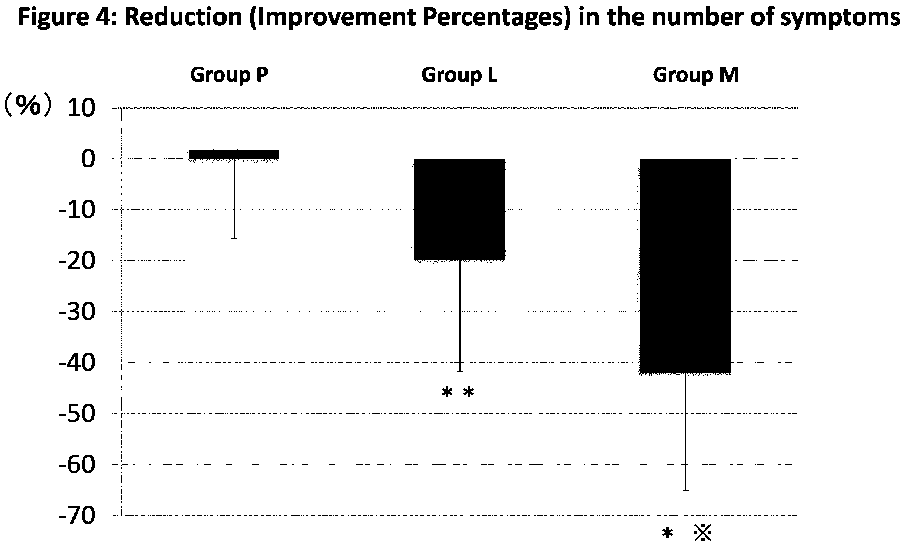

Figure 4 is a figure which shows the percentage improvement (%) in the number of symptoms in each group. The "**" represents a $p < 0.01$ as compared with Group P, the "*" represents a $p < 0.05$ as compared with Group L, and the '※' represents a $p < 0.0001$ as compared with Group P.

MODE FOR CARRYING OUT THE INVENTION

The agent for preventing or ameliorating orthostatic hypotension of the present invention comprises, as an active ingredient, a lactic acid bacterium, Lactobacillus delbrueckii subspecies lactis, or a lactic acid bacterium-derived ingredient therefrom. Therefore, the agent of the present invention can comprise only the lactic acid bacterium, Lactobacillus delbrueckii subspecies lactis, or a lactic acid bacterium-derived ingredient therefrom, whereas it can also comprise another active ingredient.

<Lactic acid bacteria>

The lactic acid bacterium, Lactobacillus delbrueckii subspecies lactis which is used in the present invention can be isolated from fermented foods such as fermented milk or cheese, or can be used after obtaining an isolated strain. As the lactic acid bacterium which is used in the present invention, one which contains a lactic acid bacterium selected from the group consisting of Lactobacillus delbrueckii subspecies lactis KLAB-4 strain (Lactobacillus delbrueckii subsp. lactis KLAB-4, hereinafter also referred to as "KLAB-4 strain") and variants thereof is preferred.

KLAB-4 strain is a strain that is described in detail in Patent Document 1 (Japanese Patent Gazette No. 5554994). It has been confirmed that, in this strain, the sequence from the 5'-terminal base to the 544th base of its 16s rRNA gene has a homology of 90% or more to the standard strain of Lactobacillus delbrueckii subsp. lactis, and therefore, it was identified as Lactobacillus delbrueckii subsp. lactis.

The lactic acid bacterium which is preferred for the use in the present invention is not only KLAB-4 strain but also its variant. Such a variant is not especially limited, and can be one which is obtained by natural mutation, one which is obtained by artificially inducing mutation by a known method such as exposure to radiation or mutagen, and the like. As long as the variant has a property (particularly, an action of preventing or ameliorating orthostatic hypotension) which is equivalent as compared to KLAB-4 lactic acid bacterium, it can be used in the present invention.

The lactic acid bacterium, Lactobacillus delbrueckii subspecies lactis, for example, KLAB-4 strain or a variant thereof can be cultured by using a known common culture medium and method. These lactic acid bacteria can be cultured by carrying out a usual lactic acid bacterium culture, using a medium in which these cells can grow, for example, MRS medium, under common culture conditions, in a container which is usually used such as a fermentation jar, a test tube, a bottle, a flask, or the like.

The lactic acid bacterium which is used in the present invention may be alive or dead. Live bacteria mean bacterial cells that are alive, and dead bacteria mean bacterial cells that were killed by treatment with heat, pressure, a drug, or the like.

Further, a lactic acid bacterium-derived ingredient which is obtained from a lactic acid bacterium can also be used in the present invention, as with the bacterial cell as long as said ingredient has a comparable function. The lactic acid bacterium-derived ingredient means a part or an ingredient of a bacterial cell, or an ingredient which contains any one of these, and may be, for example, a processed product of bacterial cells which can be obtained by applying at least one of processes such as grinding, crushing, extraction, fractionation, drying, heating, freezing, cooling, concentration, dilution, and the like to the lactic acid bacterium. The lactic acid bacterium-derived ingredient may be in any form such as liquid, paste, powder, granule, or the like. A residue which is obtained after extraction of any ingredient from the bacterial cells may also be used, and for example, a residue after extraction with hot water may be used.

Lactic acid bacterium powder of KLAB-4 strain is commercially available under the product name of "Lactic acid bacterium LAB4" (produced by Kaneka Corporation), and this can also be used.

<Water-soluble dietary fiber ingredient>

The agent for preventing or ameliorating orthostatic hypotension of the present invention can comprise a water-soluble dietary fiber ingredient in addition to the lactic acid bacterium or a lactic acid bacterium-derived ingredient therefrom as described above. It was found that the effect of the agent of the present invention can be enhanced by further comprising such an ingredient.

The water-soluble dietary fiber ingredient means a water-soluble dietary fiber, or a food or drug or a food- or drug-material containing the same. A specific example of the water-soluble dietary fiber ingredient includes, for example, a natural or synthetic water-soluble dietary fiber or water-soluble dietary fiber-containing material, such as glucomannan, pectin, guar gum, alginic acid, polydextrose, $\beta$-glucan, fructan, inulin, levan, graminan, gum arabic, maltitol, psyllium, indigestible oligosaccharide, indigestible dextrin, agarose, sodium alginate, carrageenan, fucoidan, porphyran, laminaran, seaweed and agar.

The water-soluble dietary fiber ingredient can be contained in the agent of the present invention, or can be combined with the lactic acid bacterium or a lactic acid bacterium-derived ingredient therefrom when administered or taken. Alternatively, separate preparations containing each can be administered or taken, and in such a case, both can be administered or taken simultaneously or at intervals. In other words, the agent or composition of the present invention includes not only an embodiment in which the lactic acid bacterium or a lactic acid bacterium-derived ingredient therefrom and a water-soluble dietary fiber are contained in the same composition, but also an embodiment in which they are administered or taken as separate agents or compositions simultaneously or at intervals. For example, the agent of the present invention enveloped in a capsule can be taken with a drink which comprises a water-soluble dietary fiber ingredient.

In case in which a water-soluble dietary fiber ingredient is contained in the agent of the present invention, or in case in which the agent of the present invention and a water-soluble dietary fiber ingredient are used in combination, the ratio (by weight) is not particularly specified, but for example, 0.1 to 2 parts of the water-soluble dietary fiber ingredient is preferred to 1 part of the lactic acid bacterium or a lactic acid bacterium-derived ingredient therefrom.

A mechanism with which the effect of lactic acid bacterium is enhanced by the water-soluble dietary fiber ingredient is not known, and the present invention is not bound by any specific mechanism. For example, glucomannan has a high hydrophilicity, and can absorb about 100 times by weight of water based on the weight of glucomannan. Therefore, it is thought that glucomannan absorbs water in the stomach whereby the volume of glucomannan increases to about 100 times, which leads to a condition where gastric mucosa is covered with a thin membrane. Therefore, it can be thought as one possibility that the presence of the water-soluble dietary fiber ingredient can regulate the absorption of lactic acid bacterium.

<Other ingredients>

The agent for preventing or ameliorating orthostatic hypotension of the present invention comprises the lactic acid bacterium or a lactic acid bacterium-derived ingredient therefrom as described above as the only essential ingredient, but it can comprise another active ingredient such as a water-soluble dietary fiber ingredient or it can be used in combination with another active ingredient. The composition which comprises the agent for preventing or ameliorating orthostatic hypotension of the present invention comprises one or more other ingredients in addition to the agent of the present invention.

The composition of the present invention can be a pharmaceutical composition, a food or drink composition, a feed composition for animal, a pharmaceutical composition for animal, and the like, and a specific embodiment thereof is not limited. For example, in case in which the composition of the present invention is used as a pharmaceutical composition, as a form or dosage form thereof, a capsule, a tablet, a pill, a powder preparation, a granule preparation, a drink, a syrup, an injection, a transfusion, a nasal drop, an eye drop, a suppository, a plaster, a spray and the like can be exemplified. In case in which the composition of the present invention is used as a food or drink composition, it can be in a form of general food together with various food materials or ingredients, or can be a functional food such as a supplement in a form such as a capsule or a tablet.

An arbitrary ingredient contained in the composition of the present invention can be appropriately selected depending on the property, form, process for preparation, and the like of the composition, and can be various ingredients or additives which are known in the pharmaceutical, food, or cosmetic industry, and the like. As additives which can be contained in the composition of the present invention, an excipient, a disintegrant, a lubricant, a binding agent, a surfactant, a fluidity promoter, a coloring agent, a solvent, a thickener, a dispersing agent, a pH-regulator, a moisturizer, a stabilizer, a preservative, a fragrance or flavor, and the like that are pharmaceutically acceptable, or are usually used in the pharmaceutical, food, or cosmetic industry, and the like, can be exemplified.

These additives are appropriately selected according to the desired dosage form and the like. For example, in case in which the composition of the present invention such as a pharmaceutical composition or a food or drink composition is in the form of a powdered preparation, a granular preparation, a tablet, a capsule, and the like, examples of the excipient which is used include monosaccharides or disaccharides such as lactose, sucrose, glucose, sorbitol and lactitol, starches such as corn starch and potato starch, crystalline cellulose, and inorganic substances such as light silica gel, synthetic aluminum silicate, magnesium metasilicate aluminate, calcium hydrogen phosphate, and silicon dioxide. Furthermore, a binding agent, a disintegrant, a surfactant, a lubricating agent, a fluidity promoter, an antioxidant, an aggregation preventing agent, an absorption promoter, a solubilizer, a stabilizer, a preservative, a desiccant, a coloring agent, a fragrance or flavor, and the like can be appropriately used as necessary.

Examples of the binding agent include starch, dextrin, powdered gum arabic, gelatin, hydroxypropyl starch, carboxymethyl cellulose sodium salt, methyl cellulose, crystalline cellulose, ethyl cellulose, and polyvinylpyrrolidone.

Examples of the disintegrant include starches, carboxymethyl cellulose (CMC), hydroxypropyl cellulose (HPC), carboxymethyl cellulose sodium salt, polyvinylpyrrolidone, and the like.

Examples of the surfactant include soybean lecithin, sucrose fatty acid ester, and the like; examples of the lubricant include talc, wax, sucrose fatty acid esters, hydrogenated vegetable oils, calcium stearate, magnesium stearate, and the like; and examples of the fluidity promotor include anhydrous silicic acid, dry aluminum hydroxide, magnesium silica, and the like.

The above also applies to a case in which the composition of the present invention is used as a feed for animal or a pharmaceutical composition for animal, and the composition of the present invention can be in a form or dosage form such as a feed, a supplement, a medicament, or the like as desired by using various materials for feed or pharmaceutically acceptable ingredients.

The agent or composition of the present invention may be administered or used alone or may be used in combination with other agent(s). Furthermore, in a case in which the agent or composition of the present invention is used in combination with other agent(s), the two may be used simultaneously or may be used one after the other.

The route of administration (or intake or application) of the agent or composition for preventing or ameliorating orthostatic hypotension of the present invention can be appropriately selected from, for example, oral, transdermal, transintestinal, intrarectal administration route, and the like. The amount of administration (or intake or application) per day of the agent or composition of the present invention which is effective for prevention or amelioration of orthostatic hypotension varies depending on the form of the preparation, the method or route of administration and the like, the age and body weight of the subject, the severity of the disease and the like; for example, in case of oral route, in the human being, generally about 0.1 mg to about 1000 mg/kg body weight/day, preferably about 1 mg to about 500 mg/kg body weight/day, and most preferably about 2 mg to about 300 mg/kg body weight/day, as the amount of the effective ingredient that is a lactic acid bacterium or a lactic acid bacterium-derived ingredient, can be administered or taken all at once or in divisions. The timing of administration or intake is not particularly specified, and for example, can be simultaneous with a meal, just after a meal, just before a meal, before bedtime, or the like.

Since the agent or composition of the present invention is highly safe, it can be administered to not only patients but also healthy subjects. Further, the agent or composition of the present invention can be administered (or taken or applied) similarly to subjects, specifically human being and non-human animals, preferably mammals. Examples of the non-human animals include domestic animals such as cow, horse, pig and sheep, and companion animals such as dog and cat. The amount of administration can be adjusted depending on the characteristics of the animal, using the above amount as the standard. For example, in case of a small animal, the amount of administration or intake can be adjusted and given so that it corresponds to about 0.01 to about 1000 mg/kg body weight /day, more preferably about 0.1 to about 30 mg/kg body weight/day as the amount of the lactic acid bacterium or a lactic acid bacterium-derived ingredient therefrom.

Whether orthostatic hypotension has been ameliorated or not by the agent or composition of the present invention can be evaluated by determining whether the number of symptoms has increased or decreased and/or whether the results of the Head-Up Tilf test have been improved or not at a later time point as compared to an earlier time point out of different time points such as before and after the administration or intake, or during the administration or intake, or after the administration or intake.

EXAMPLES

Hereinafter, the present invention will be more specifically described by way of Examples, but the present invention is not limited by these Examples.

1. Production of capsule preparation

As a lactic acid bacterium Lactobacillus delbrueckii subspecies lactis, KLAB-4 lactic acid bacterium powder (product name: "Lactic acid bacterium LAB4" (manufactured by Kaneka Corporation)), and as a water-soluble dietary fiber ingredient, glucomannan (product name: "RHEOLEX RS" (manufactured by Shimizu Chemical Corporation) were used, and the following 3 kinds of capsule preparations were produced.

Production example 1:

One hundred mg of the lactic acid bacterium powder, 50 mg of glucomannan powder, 17.5 mg of crystalline cellulose powder, 2 mg of calcium stearate powder, and 0.5 mg of silicone dioxide powder per 1 capsule were mixed and stirred until uniform, and then filled into a commercially available capsule (product name: "Pig Gelatin Clear No. 3" (manufactured by Qualicaps Co., Ltd.).

Production example 2:

One hundred mg of the lactic acid bacterium powder, 50 mg of lactose, 17.5 mg of crystalline cellulose powder, 2 mg of calcium stearate powder, and 0.5 mg of silicone dioxide powder per 1 capsule were mixed and stirred until uniform, and then filled into the same commercially available capsule as that used in Production example 1.

Comparative example:

The same capsule preparation as in Production example 1 was produced except that it contained 150 mg of lactose in place of the lactic acid bacterium powder and glucomannan powder.

These 3 kinds of capsule preparations did not have a difference in the appearance, taste and smell, and were not distinguishable.

2. Study on the effect on orthostatic hypotension

The present study was carried out after sufficient informed consent was obtained upon obtaining the approval from the ethical committee of a public interest incorporated foundation, the International Foundation of Comprehensive Medicine (2017).

<Subjects>

Forty-five patients who had been diagnosed as orthostatic hypotension (25 men and 20 women) were divided into 3 groups of placebo group (P), lactic acid bacterium group (L) and lactic acid bacterium + glucomannan group (M) by an enveloped method to investigate the effect of the agent of the present invention by a double blind method.

There was no statistically significant difference in the age and the sex of the subjects among the groups. Before the start of administration, the $\Delta$SBP of each group was 25.5 $\pm$ 5.1 in Group P, 28.4 $\pm$ 4.1 in Group L, and 27.7 $\pm$ 4.4 in Group M; The number of symptoms was 3.8 $\pm$ 0.68 in Group P, 3.7 $\pm$ 0.73 in Group L, and 4.1 $\pm$ 0.77 in Group M. Again there was no statistically significant difference in these. The details of the subjects constituting each group are shown in Table 1.

Table 1: Details of subjects of each group

|  | Men | Women | Total | Age | $\triangle$SBP | Number of symptoms |
|---|---|---|---|---|---|---|
| Group P | 8 | 7 | 15 | 38.2 $\pm$ 7.7 | 25.5 $\pm$ 5.1 | 3.8 $\pm$ 0.68 |
| Group L | 8 | 6 | 14 | 42.1 $\pm$ 9.9 | 28.4 $\pm$ 4.1 | 3.7 $\pm$ 0.73 |
| Group M | 9 | 7 | 16 | 43.4 $\pm$ 12.3 | 27.7 $\pm$ 4.4 | 4.1 $\pm$ 0.77 |
| Total | 25 | 20 | 45 | 39.9 $\pm$ 11.0 |  |  |

Group P, Group L, and Group M took 3 capsules of Comparative example, Production example 2, and Production example 1, respectively, per day (1 capsule at a time) immediately after meals. The administration period was 3 months.

<Evaluation of orthostatic hypotension>

Before and after the start and end of administration, the presence or absence of improvement in orthostatic hypotension was evaluated by the following methods.

(1) In the Head-Up Tilt test, blood pressure was measured three times every 2 minutes after 10 minutes of resting supine position, followed by loading 10 minutes of standing.
(2) $\triangle$SBP (systolic blood pressure, mmHg), which is SBP after 10 minutes in the standing position - SBP at 3 minutes in the supine position, is calculated.
(3) The number of existing symptoms of the five major symptoms of orthostatic hypotension (1. orthostatic dizziness and vertigo, 2. discomfort after standing, 3. discomfort after bathing, 4. palpitations and shortness of breath, and 5. difficulty getting up in the morning) is counted.

The statistical figures were expressed as an average value $\pm$ standard deviation, and analyzed by a statistic software SSMC, MAC version.
The results are shown in Figures 1 to 4.
Figure 1 shows a comparison of the changes in differences of standing SBP minus supine SBP ($\triangle$SBP) measured before and after the administration. There was no significant difference in the values among the groups before administration. For Group P, there was no change in the value after administration from that before administration. On the other hand, for Groups L and M, the differences were significantly reduced after administration compared to before administration (**: $p<0.01$).
Figure 2 is a figure which compares the percentages of improvement of $\triangle$SBP for each group. The improvement percentage was calculated as follows:

```
Improvement percentage (%) = [(△SBP after administration - △SBP
before administration) / △SBP before administration] x 100
```

As shown in Figure 2, there were significant differences between Group P and Group L, as well as Group P and Group M (**: $p<0.01$). On the other hand, there was no significant difference between Group L and Group M.
Figure 3 compares changes in the number of symptoms before and after administration. Before administration, there was no significant difference in the number of symptoms among the groups. After administration, there was no significant difference in the number of symptoms in Group P compared to before administration, but in Groups L and M the number of symptoms was significantly reduced compared to before administration. In addition, a comparison between Group L and Group M before and after administration showed that the reduction in Group M (***: $p<0.001$) was remarkable and more significant than that in Group L (**: $p<0.01$).
Figure 4 compares the reduction in the number of symptoms (improvement percentage) for each group. The improvement percentage for the number of symptoms was calculated as follows:

```
Improvement percentage (%) = [(value after administration - value
before administration) / value before administration] x 100
```

For Group P, there was almost no difference between before and after administration, whereas Group L (**:

p<0.01) and Group M (※: p<0.0001) improved significantly relative to Group P. The improvement percentage in Group M was very remarkable and also significantly improved even when compared to Group L (*: p<0.05).

<Investigation of safety>

In each group, blood and urine specimens were collected before the start and after the end of the administration, and complete blood count (red blood cell, white blood cell, hemoglobin, hematocrit, and platelet), Alb, GOT, GPT, LDH, AIP, γ-GTP, amylase, BUN, creatinine, blood sugar, hemoglobin A1c, and urine (protein, sugar, urobilinogen) were tested to compare the results.

Any group did not exhibit any abnormalities in the blood and urine tests before and after the administration. As a side effect, loose stool was observed in 2 cases in Group L, and in 1 case in Group M.

According to the results of the present study, orthostatic hypotension was improved in both of Group L and Group M that were administered the agent for preventing or ameliorating orthostatic hypotension of the present invention. That is, treatments in Group L and Group M were significantly effective to orthostatic hypotension compared to Group P. Comparing Group L and Group M, Group M was more effective. As the side effects, slight soft stool was observed in a few subjects in Group L and Group M, which demonstrated that the agent or composition of the present invention is highly safe.

The present application is based on the Japanese Patent Application No. 2019-218216 filed on December 2, 2019, and the subject matters described in the specification and the scope of the claims for patent of Japanese Patent Application No. 2019-218216 are all incorporated in this specification.

**Claims**

1. An agent for preventing or ameliorating orthostatic hypotension comprising, as an active ingredient, a lactic acid bacterium Lactobacillus delbrueckii subspecies lactis or a lactic acid bacterium-derived ingredient therefrom.

2. The agent for preventing or ameliorating orthostatic hypotension according to claim 1, wherein said lactic acid bacterium comprises a lactic acid bacterium selected from the group consisting of Lactobacillus delbrueckii subspecies lactis KLAB-4 strain (NITE BP-394) and variants thereof.

3. The agent for preventing or ameliorating orthostatic hypotension according to claim 1 or 2, which further comprises a water-soluble dietary fiber ingredient, or which is used in combination with a water-soluble dietary fiber ingredient.

4. The agent for preventing or ameliorating orthostatic hypotension according to claim 3, wherein said water-soluble dietary fiber ingredient is selected from the group consisting of glucomannan, pectin, guar gum, alginic acid, polydextrose, β-glucan, fructan, inulin, levan, graminan, gum arabic, maltitol, psyllium, indigestible oligosaccharide, indigestible dextrin, agarose, sodium alginate, carrageenan, fucoidan, porphyran, laminaran, seaweed, and agar.

5. The agent for preventing or ameliorating orthostatic hypotension according to claim 4, wherein said water-soluble dietary fiber ingredient is glucomannan.

6. A composition comprising the agent for preventing or ameliorating orthostatic hypotension according to any one of claims 1 to 5.

7. A pharmaceutical composition comprising the agent for preventing or ameliorating orthostatic hypotension according to any one of claims 1 to 5.

8. A food or drink composition comprising the agent for preventing or ameliorating orthostatic hypotension according to any one of claims 1 to 5.

9. A feed composition for animal or a pharmaceutical composition for animal comprising the agent for preventing or ameliorating orthostatic hypotension according to any one of claims 1 to 5.

10. A method for preventing or ameliorating orthostatic hypotension, comprising administering the agent for preventing or ameliorating orthostatic hypotension according to any one of claims 1 to 5, or the composition according to any one of claims 6 to 9, to a subject.

Figure 1: Changes in ΔSBP for each group before and after administration

# Figure 2: Improvement Percentages in ΔSBP for each group

# Figure 3: Changes in the number of symptoms

EP 4 070 806 A1

# Figure 4: Reduction (Improvement Percentages) in the number of symptoms

## INTERNATIONAL SEARCH REPORT

| International application No. |
|---|
| PCT/JP2020/044053 |

### A. CLASSIFICATION OF SUBJECT MATTER

Int.Cl. A61K35/747(2015.01)i, A23L33/135(2016.01)i, A61K31/702(2006.01)i, A61K31/716(2006.01)i, A61K31/721(2006.01)i, A61K31/729(2006.01)i, A61K31/731(2006.01)i, A61K31/732(2006.01)i, A61K31/733(2006.01)i, A61K31/734(2006.01)i, A61K31/736(2006.01)i, A61K36/02(2006.01)i, A61P9/02(2006.01)i, A61P43/00(2006.01)i
FI: A61K35/747, A61K31/716, A61K31/732, A61K31/736, A61K31/733, A61K31/734, A61K31/721, A61K31/731, A61K31/729, A61K31/702, A61K36/02, A61P9/02, A61P43/00121, A23L33/135

According to International Patent Classification (IPC) or to both national classification and IPC

### B. FIELDS SEARCHED

Minimum documentation searched (classification system followed by classification symbols)
Int.Cl. A61K35/747, A23L33/135, A61K31/702, A61K31/716, A61K31/721, A61K31/729, A61K31/731, A61K31/732, A61K31/733, A61K31/734, A61K31/736, A61K36/02, A61P9/02, A61P43/00

Documentation searched other than minimum documentation to the extent that such documents are included in the fields searched
Published examined utility model applications of Japan      1922–1996
Published unexamined utility model applications of Japan    1971–2020
Registered utility model specifications of Japan            1996–2020
Published registered utility model applications of Japan    1994–2020

Electronic data base consulted during the international search (name of data base and, where practicable, search terms used)
CAplus/MEDLINE/EMBASE/BIOSIS/WPIDS(STN)

### C. DOCUMENTS CONSIDERED TO BE RELEVANT

| Category* | Citation of document, with indication, where appropriate, of the relevant passages | Relevant to claim No. |
|---|---|---|
| Y | WO 2008/120713 A1 (SUNTORY LTD.) 09 October 2008 (2008-10-09), claim 1, paragraphs [0004], [0046], [0052], examples 1, 2, 4 | 1–10 |
| Y | WO 2009/066681 A1 (KANEKA CORPORATION) 28 May 2009 (2009-05-28), abstract | 1–10 |
| Y | NAKAMURA, Sadako et al., Daily feeding of fructooligosaccharide or glucomannan delays onset of senescence in SAMP8 mice, Gastroenterol. Res. Pract., 02 June 2014, vol. 2014, article ID 303184, pp. 1–11, title, abstract, fig. 1–7 | 3–5 |

☐ Further documents are listed in the continuation of Box C.      ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 December 2020 | 12 January 2021 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

Form PCT/ISA/210 (patent family annex) (January 2015)

| INTERNATIONAL SEARCH REPORT | International application No. |
|---|---|
| Information on patent family members | PCT/JP2020/044053 |

| | | |
|---|---|---|
| WO 2008/120713 A1 | 09 October 2008 | US 2010/0150891 A1<br>claim 1, paragraphs [0004], [0048],<br>[0054], examples 1, 2, 4 |
| WO 2009/066681 A1 | 28 May 2009 | US 2010/0278795 A1<br>abstract |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5554994 B
- JP 6747724 B

- JP 2019218216 A

**Non-patent literature cited in the description**

- **AIRO TATEGAKI.** Health functions of lactic acid bacteria (Lactic Acid Bacteria and Human Health). *Comprehensive Medicine,* 2018, vol. 17 (1), 8-19